# EUROPEAN PATENT APPLICATION

(11) **EP 3 978 057 A1**
(43) Date of publication of application: **06.04.2022**
(21) Application number: 21200750.4
(22) Date of filing: 04.10.2021
(51) Int. Cl.: A61M 16/10, A61M 16/16, A61M 16/00, A61M 16/08, A61M 16/20

(54) **VENTILATION SYSTEM HAVING MULTIPLE VENTILATION MODES CONTROLLED BY A DISPOSABLE CONTROL UNIT**

(30) Priority: 05.10.2020 US 202063087354 P; 03.05.2021 US 202117306370
(71) Applicant: Biosense Webster (Israel) Ltd, Yokneam, 2066717 (IL)
(72) Inventor: GOVARI, Assaf, 2066717 Yokneam (IL); ALGAWI, Yehuda, 2066717 Yokneam (IL); AHARON, Eran, 2066717 Yokneam (IL)
(74) Representative: Carpmaels & Ransford LLP

(57) **Abstract**

The present invention is directed to a patient ventilation system, method and software product comprising a controller, which is configured, based on received signals, to control one or more valves, pressure regulators and flow regulators of the system and to set, monitor and control various parameters relating to at least one of the flow rate and the pressure of oxygen-enriched humidified air (OHA) being provided to the lungs of a patient.

## Description

### CROSS-REFERENCE TO RELATED APPLICATIONS

This application is related to and claims priority to U.S. Provisional Patent Application 63/087,354, filed on October 5, 2020, whose disclosure is incorporated herein by reference.

### FIELD OF THE INVENTION

The present invention relates generally to ventilation systems, and particularly to ventilation systems having multiple ventilation modes controlled by a disposable control unit, and methods for producing the same.

### BACKGROUND OF THE INVENTION

Various types of ventilation systems, which may be used for addressing respiratory distress in patients, have been described in the patent literature.

For example, U.S. Patent 10,092,721 describes a breathing circuit including a flexible hollow tube having a generally circular cross section and a flexible printed circuit board assembly disposed in the flexible hollow tube. The flexible printed circuit board assembly defines part of at least one inspiratory passage within the flexible hollow tube and part of at least one expiratory passage within the flexible hollow tube. The flexible printed circuit board assembly is configured to heat airflow within one or more of the at least one inspiratory passage and the at least one expiratory passage, and monitor a first property of the airflow within one or more of the at least one inspiratory passage and the at least one expiratory passage at multiple locations along the axial length of the flexible hollow tube.

U.S. Patent 8,714,156 describes a ventilator system that addresses respiratory distress due to the onset of an epidemic or pandemic disease state. In particular, a ventilator system that can be manufactured quickly with minimal skill requirements and employed rapidly in response to epidemic respiratory disease conditions.

U.S. Patent Application Publication 2017/0304570 describes a high flow therapy system for delivering heated and humidified respiratory gas to an airway of a patient. The system includes a respiratory gas flow pathway for delivering the respiratory gas to the airway of the patient by way of a non-sealing respiratory interface, wherein flow rate of the pressurized respiratory gas is controlled by a microprocessor.

### SUMMARY OF THE INVENTION

Ventilation systems are used for mechanically assisting or replacing autonomic breathing when a patient cannot breathe by himself/herself adequately. Ventilator components of a ventilation system typically operate in an oxygen-enriched atmosphere where the presence of any oil or grease could be catastrophic. Therefore, such components are typically difficult to adequately clean and sterilize and typically increases the cost of the ventilation system.

An embodiment of the present invention that is described herein provides a ventilation system comprising a substrate, one or more sensors, one or more valves, pressure regulators and flow regulators, and a controller. The ventilation system further comprises an air compressor, which is configured to compress a preassigned gas mixture of oxygen-enriched humidified air (OHA) to a preassigned pressure, and a humidifier, which is configured to set a preassigned humidity of the OHA.

In some embodiments, the ventilation system comprises (i) an OHA tube, which is configured to flow the OHA produced by the compressor and humidifier toward lungs of a patient, (ii) an exhaust tube, which is configured to flow exhaust air exhaled from the lungs to an air evacuation system of a hospital, and (iii) an intubation tube, which is configured to flow the OHA and the exhaust air between the lungs and the OHA and exhaust tubes. The intubation tube comprising a distal end, configured to be inserted into a trachea of the patient, and a proximal end, configured to be connected to the OHA and exhaust tubes. In the context of the present disclosure and in the claims, the term "exhaust air" refers to air flowing from the lungs into the air evacuation system of the hospital. Note that the term "exhaled" is applicable when the patient can exhale the air from the lungs, otherwise, the term exhaled may be replaced with a term "sucked" referring to suctioning, typically part of the air, from the patient lungs.

In some embodiments, one or more of the sensors, valves, pressure regulators and flow regulators are incorporated into the substrate, which is coupled to the OHA and exhaust tubes. The substrate may comprise a printed circuit board (PCB) or any other suitable substrate having electrical traces, which are configured to electrically connect between the (i) one or more sensors, valves, pressure regulators and flow regulators, and (ii) the controller.

In some embodiments, the one or more sensors are configured to output a signal indicative of at least the flow rate and gas mixture composition of the OHA and exhaust air, and of the humidity-level of the OHA. The sensors may comprise various types of sensors, such as but not limited to a flow sensor, an oxygen concentration sensor, a carbon dioxide concentration sensor, a humidity sensor, and a temperature sensor. In the context of the present disclosure and in the claims, the term "concentration sensor" refers to a sensor configured to measure the percentage of a gas molecule (e.g., oxygen or carbon dioxide) within a given volume of fluid, such as the aforementioned OHA and exhaust air.

In some embodiments, the one or more valves, pressure regulators and flow regulators comprise electromagnets and/or membranes, and are configured to set the flow rate of at least the OHA into the patient lungs. In some embodiments, at least one of the valves comprises a fail-safe valve, which is configured, in response to a failure in at least one of the flow regulators and the pressure regulators, to limit at least one of the flow rate and pressure of the OHA.

In some embodiments, the compressor and humidifier are reusable for ventilating multiple patients sequentially, whereas (i) the PCB having the sensors, valves, pressure regulators, flow regulators, and controller, and (ii) the tubes are designed to be disposable parts. The disposable parts are trashed after ventilating a first patient and a new set of the disposable parts are used for ventilating a second patient. Note that the disposable parts are not being sterilized, and therefore, can be made from materials having reduced cost and have a compact design. In the present example, control unit 33 may have a size of a laptop computer or any other suitable size, and may have a cost significantly smaller than a cost of a reusable control unit (not shown) having the same functionality.

In some embodiments, the controller is configured, based on the signal, to control the gas mixture, the flow rate and the humidity of the OHA flowing into the lungs. The controller is configured to control: (i) the air compressor and the humidifier by setting the preassigned gas mixture, pressure and humidity, and (ii) the one or more valves, pressure regulators, and flow regulators by setting the flow rate of at least the OHA.

In some embodiments, the controller is configured to display, on a display, one or more parameters indicative of at least the flow rate of at least the OHA. For example, the controller is configured to display the oxygen concentration, humidity-level and flow rate of the OHA.

In some embodiments, based on one or more signals indicative of: (i) the concentration of carbon dioxide and/or oxide of exhaust air exhaled from the patient lungs, and (ii) flow rate of the exhaust air, the controller is configured to switch between a normal-ventilation mode (NVM) and a hyper-ventilation mode (HVM) of the ventilation system.

In the context of the present disclosure and in the claims, the term NVM refers to about 15 breathing cycles per minute, and the term HVM refers to any suitable larger number of breathing cycles per minute, such as but not limited to, between about 100 and 300 breathing cycles per minute. Additionally or alternatively, the controller is configured to operate in other modes, such as in a breathing rate larger than the NVM and smaller than the HVM.

In some embodiments, the controller is configured to switch between the NVM and HVM modes by controlling the frequency at which the compressor and humidifier are producing the specified attributes of the OHA.

The disclose techniques improve the efficiency of intensive care units (ICUs) and operating theatres by reducing the size and cost of ventilation systems and by eliminating the need to sterilize parts of the ventilation system that may be contaminated during the ventilation process of a patient.

Another embodiment of the present invention is directed to a computer software product, comprising a non-transitory computer-readable medium in which program instructions are stored, which instructions, when read by a controller for a ventilator system for a patient, the controller programmed to perform the steps of: setting preassigned thresholds for at least one of the gas mixture, the flow rate and the humidity of an oxygen-enriched humidified air (OHA) to be directed to the lungs of the patient; receiving one or more of the signals from sensors of the system, to control the gas mixture, the flow rate and the humidity of the OHA directed into lungs of the patient; displaying on a display of the system, one or more parameters indicative of at least one of the flow rate, concentration levels of the gas mixture or humidity of the OHA being directed into lungs of the patient.

The computer software product further comprises performing the steps of: receiving one or more of the signals from sensors of the system, to monitor expiration from the lungs of the patient.

In some embodiments, the computer software product further comprises performing the steps of: setting a preassigned threshold for the temperature of the OHA to be directed to the lungs of the patient; and receiving one or more of the signals from sensors of the system, to monitor the temperature of expiration from the lungs of the patient.

In some embodiments, the computer software product comprises performing the steps of: setting preassigned thresholds for at least one of the gas mixture having oxygen percentage between about 21% and 100%, the flow rate pressure between about 0 Cm and 100 Cm, and the humidity of the OHA between about 40% and 100%.

Additionally, in some embodiments according to the present invention, the computer software product further comprises performing the steps of: switching between a normal-ventilation mode (NVM) and a hyper-ventilation mode (HVM) for the ventilation, wherein the NVM comprises about 15 breathing cycles per minute, and the HVM comprises between about 100 and 300 breathing cycles per minute.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a ventilation system, in accordance with an embodiment of the present invention; and
Fig. 2 is a flow chart that schematically illustrates a method for producing or configuring a ventilation system, in accordance with an embodiment of the present invention.

### DETAILED DESCRIPTION OF EMBODIMENTS

### OVERVIEW

Ventilation systems are used for mechanically assisting or replacing autonomic breathing when a patient cannot breathe by himself/herself adequately. Ventilator components of a ventilation system typically operate in an oxygen-enriched atmosphere where the presence of any oil or grease could be catastrophic. Therefore, such components are typically difficult to adequately clean and sterilize and typically increases the cost of the ventilation system.

### SYSTEM DESCRIPTION

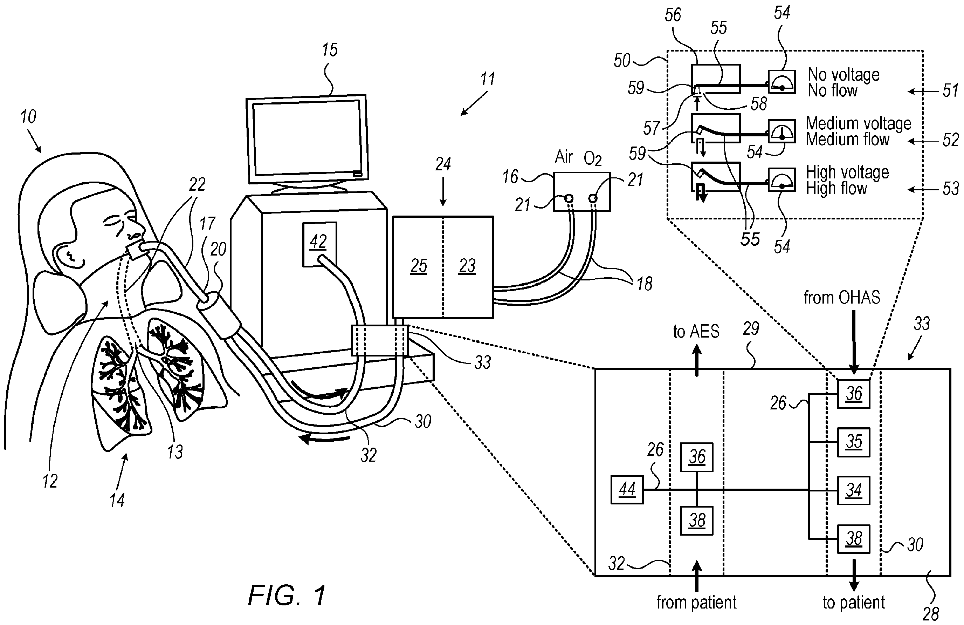

Fig. 1 is a schematic, pictorial illustration of a ventilation system 11, in accordance with an embodiment of the present invention.

In some embodiments, ventilation system 11 comprises an oxygen-enriched humidified air (OHA) supply subsystem (OHAS) 24 comprising an air compressor 23 and a humidifier 25. Air compressor 23 is configured to compress a preassigned gas mixture to a preassigned pressure described below.

In some embodiments, ventilation system 11 comprises tubes 18 connected between OHAS 24 and a hospital supply 16. In the present example, air compressor 23 is configured to receive, via outlets 21 of hospital supply 16, oxygen (O₂) and air, so as to produce the aforementioned OHA at a preassigned mixture (e.g., defined in volumetric percentage) and preassigned pressure. In some embodiments, the OHA has a typical percentage of oxygen between about 21% and 100% and a preassigned pressure zero CmH₂O (when a patient 10 is self-breathing) and 50 CmH₂O.

In some embodiments, humidifier 25 is configured to moisture) harvest from the environment or to receive water from any other suitable source, so as to set a preassigned humidity of the OHA. For example, humidifier 25 is configured to supply OHA at a selected humidity level between about 40% and 100%, or at any other suitable humidity. In the present example, OHAS 24 comprises reusable air compressor 23 and humidifier 25, which may have to undergo a minimal or no cleaning or sterilization between ventilations of different patients 10.

In some embodiments, system 11 comprises an intubation tube 22 having a distal end 13, which is configured to be inserted into a trachea 12 of patient 10, and a proximal end 17, which is connected to a bifurcation adaptor 20.

In some embodiments, ventilation system 11 comprises an OHA tube 30, which is coupled between bifurcation adaptor 20 and OHAS 24, and is configured to flow the OHA produced by air compressor 23 and humidifier 25 or OHAS 24, via intubation tube 22, toward lungs 14 of patient 10. Note that lungs 14 are exposed by depiction in Fig. 1 and shown for the sake of more clear presentation, noting that lungs 14 in the thoracic cavity of the patient 10 which is covered with natural tissue (e.g., pleura, bone, muscles, skin, etc.) removed from Fig. 1 for the sake of conceptual clarity.

In some embodiments, system 11 further comprises an exhaust or expiration tube 32, which is coupled between bifurcation adaptor 20 and an air evacuation system (AES) 42 of the hospital. Exhaust or expiration tube 32 is configured to flow exhaust air (expiration) exhaled from lungs 14 of patient 10, via intubation tube 22, toward AES 42.

Note that the term "exhaled" is applicable when patient 10 can independently exhale at least a portion of the air from lungs 14. Otherwise, the term exhaled may be replaced with the terms "expiration", "evacuated", "aspirated" or "sucked" referring to expiration, evacuation, aspiration or suctioning, typically part of the air, from lungs 14 of patient 10.

In some embodiments, ventilation system 11 comprises a control unit 33, which is configured to monitor and control parameters of the ventilation process, such as but not limited to the gas mixture (e.g., oxygen), flow rate and the humidity of the OHA flowing into lungs 14.

Reference is now made to an inset 29 for a detailed description of control unit 33. In some embodiments, control unit 33 comprises a substrate 28, one or more sensors 38, one or more valves, such as but not limited to a fail-safe valve (FSV) 34, one or more flow regulators 36, one or more pressure regulators 35, which are all described in detail below, and a controller 44.

In some embodiments, air compressor 23 is controlled by controller 44 to supply a typical pressure between about zero CmH₂O (when a patient 10 is self-breathing) and 50 CmH₂O, as described in detail below.

In some embodiments, one or more of sensors 38, FSV 34, pressure regulators 35 and flow regulators 36, are incorporated into substrate 28, which is coupled to OHA tube 30 and exhaust tube 32.

In some embodiments, substrate 28 may comprise a printed circuit board (PCB) or any other suitable substrate having electrical traces 26, which are configured to electrically connect between the (i) sensors 38, FSV 34, pressure regulator 35 and flow regulators 36, and (ii) controller 44.

Reference is now made to an inset 50 showing an implementation of flow regulator 36. In some embodiments, flow regulator 36 comprises a housing 56, typically made from any suitable rigid polymer (e.g., acrylonitrile butadiene styrene). The housing may have two or more openings, such as openings 57 and 58. Flow regulator 36 further comprises an actuator 55, which in the present example comprises a piezoelectric actuator, and a gasket 49 coupled to the actuator. Actuator 55 is typically made from any suitable piezoelectric ceramic material, and gasket 59 is typically made from any suitable polymer, such as a suitable rubber. In the context of the present disclosure and in the claims, an element comprising a combination of actuator 55 and gasket 59 is also referred to as a membrane implemented in at least one of FSV 34, flow regulators 36, and pressure regulators 35, as will be described in detail herein.

In some embodiments, flow regulator 36 is controlled by controller 44, and in the present example has three operational states (OSs) 51, 52, and 53.

In some embodiments, at OS 51, controller 44 controls a power supply unit (PSU) 54 to shut down or disconnect the power supply to actuator 55. As a result, actuator 55 remains flat and gasket 59 blocks flow of the OHA into housing 56 through opening 57. Thus, at OS 51, flow regulator 36 does not enables flow of OHA to patient 10.

In some embodiments, at OS 52, controller 44 controls PSU 54 to provide a power supply of a medium voltage level (e.g., between about 10 V and 120 V) to actuator 55, which in response bends to some extent, so as to enable flow of the OHA into housing 56 through opening 57, followed by OHA flowing out of housing 56 through opening 59. Thus, at OS 52, flow regulator 36 enables medium flow of the OHA (e.g., at a flow rate between about 10 liter per minute (LPM) and 60 LPM, or at any other suitable flow rate) to patient 10.

In some embodiments, at OS 53, controller 44 controls PSU 54 to provide a power supply of a high voltage level (e.g., between about 120 V and 200 V) to actuator 55, which in response bends substantially so as to enable flow of the OHA through openings 57 and 58 at a flow rate between about 60 LPM and 90 LPM, or at any other suitable flow rate, to patient 10.

In some embodiments, flow regulators 36 are coupled to tubes 30 and 32, and are electrically connected to controller 44 via electrical traces 26 described below. In other embodiments, control unit 33 may have a reduce configuration comprising only one flow regulator 36 coupled to OHA tube 30. In this reduced configuration the air exhaled by patient 10 is not regulated.

In the present example, flow regulator 36 comprises a proportional 3/3-way valve, such as a piezo valves VEMP product, supplied by Festo Corporate (Esslingen am Neckar, Germany). In other embodiments, flow regulator 36 may be implemented using any other suitable elements and techniques, such as but not limited to electromagnetic or pneumatic actuators. Moreover, instead of or in addition to the aforementioned membrane, flow regulator 36 may comprise one or more electromagnets or any other suitable devices or components configured for regulating the flow of the OHA. Note that flow regulator 36 is configured to operate only in one of OSs 51-53 at the same time, so that OSs 51-53 are presented together in inset 50 for the sake of conceptual clarity.

Reference is now made back to inset 29. In some embodiments, pressure regulator 35 is coupled to OHA tube 30 and is electrically connected to controller 44 via electrical traces 26. In the present example, pressure regulator 35 comprises two membranes (such as the membranes of piezo valves VEMP product described above) and two pressure sensors arranged in a configuration, which is controlled by controller 44 for controlling the OHA pressure between about 0 CmH₂O and 50 CmH₂O as described above. In the present example, pressure regulator 35 is implemented in control unit 33 using a suitable pressure regulator selected from the VEAB series of products supplied by Festo Corporate. In other embodiments, however, pressure regulator 35 may comprise any other suitable product using any other suitable technique.

In some embodiments, control unit 33 comprises one FSV 34, which is coupled to OHA tube 30 downstream relative to flow regulator 36 and pressure regulator 35. The control unit is configured to limit the pressure of OHA flowing to patient 10, so as to prevent a barotrauma, caused by supplying overpressure of OHA to lungs 14 of patient 10. In the context of the present disclosure and in the claims, the term "fail-safe valve" refers to a valve configured to limit at least one of the pressure and flow rate of a fluid (e.g., OHA) flowing into lungs 14. In some cases, a failure may occur in at least one of flow regulator 36 and pressure regulator 35. In such events, the fail-safe valve is configured to prevent overflow and/or overpressure of OHA supplied to lungs 14.

In some embodiments, FSV 34 may comprise any suitable type of valve, such as an LRP-1/4-0.7 mechanical fail-safe valve, supplied by Festo Corporate, wherein the fraction number term "1/4" refers to the fitting size and the parameter term "0.7" refers to 0.7 Bar, which is the maximal pressure of the LRP-1/4-0.7 product. In the present example, the threshold pressure of the LRP-1/4-0.7 is adjusted to about 50 mBar, which is equivalent to the aforementioned maximal pressure of 50 CmH₂O that is allowed to be supplied to patient 10.

Note that a mechanical fail-safe valve is one type of a valve suitable for FSV 34 (e.g., a pressure relief valve, or a check valve) in accordance with embodiments of the present invention. In other embodiments, one or more of flow regulators 36, pressure regulator 35 and FSV 34 may be based on any suitable techniques other than mechanicalbased (e.g., electromechanical, electromagnetic, etc.)

In the present example, sensors 38 and flow regulators 36 are coupled to both tubes 30 and 32 and pressure regulator 35 and FSV 34 are coupled solely to OHA tube 30, but in other embodiments, control unit 33 may have any other suitable configuration. For example, sensors 38, FSV 34, and flow regulators 36 are coupled to OHA tube 30 (shown in dashed lines), whereas only one or more sensors 38 may be coupled to exhaust tube 32 (shown in dashed lines) for monitoring the exhaust air as will be described in detail below.

In some embodiments, sensors 38 are configured to output a signal indicative of at least the flow rate and gas mixture composition of the OHA and exhaust air, and of the humidity-level of the OHA. Sensors 38 may comprise one or more types of sensors, such as but not limited to a flow sensor, an oxygen concentration sensor, a carbon dioxide (CO₂) concentration sensor, a humidity sensor, and a temperature sensor. Note that the term "concentration sensor" refers to a sensor configured to measure the percentage of a gas molecule (e.g., oxygen consumed by patient 10 or carbon dioxide exhaled from lungs 14 of patient 10) within a given volume of fluid, such as the aforementioned OHA and exhaust air.

In some embodiments, one or more of FSV 34, pressure regulator 35, and flow regulators 36 comprise at least one of an electromagnet and a membrane. For example, a combination of actuator 55 and gasket 59 may serve as a membrane in flow regulators 36.

In some embodiments, control unit 33, and tubes 22, 30 and 32 are designed to be disposable components. The disposable components are discarded after first patient use, i.e. upon completion of ventilating a first patient, such as patient 10 and a new set of the aforementioned disposable components can be used for ventilating a subsequent second patient (not shown). Note that the disposable components/parts are not being sterilized, and therefore, are made from materials having reduced cost, such as the materials described in flow regulator 36.

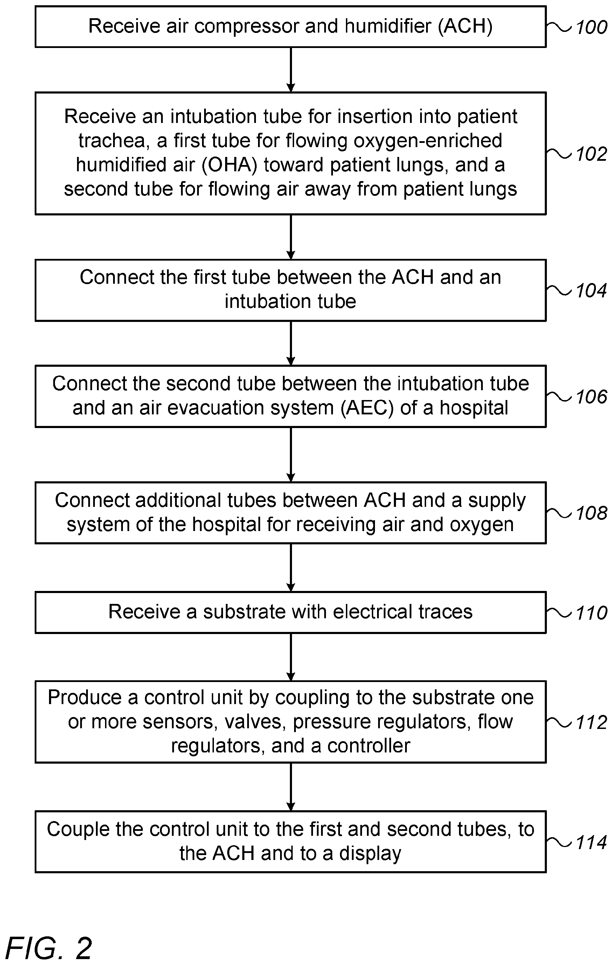

In some embodiments, one or more of FSV 34, pressure regulator 35 and flow regulators 36 of system 11 may be used in an oxygen-enriched atmosphere after being cleaned and qualified using a procedure described in detail in Fig. 2 below. Such components may undergo operative qualification testing, and subsequently, may be used as disposable components or parts of system 11.

In some embodiments, controller 44 is configured, based on one or more of the signal(s) received from sensors 38, to control the gas mixture, the flow rate and the humidity of the OHA flowing into lungs 14.

In some embodiments, controller 44 is configured to control air compressor 23 and humidifier 25 of OHAS 24, by setting the preassigned thresholds, such as gas mixture having oxygen percentage between about 21% and 100%, pressure between about 0 Cm and 100 Cm, and humidity between about 40% and 100%. Controller 44 is further configured to control one or more of FSV 34, pressure regulator 35 and flow regulators 36 by setting thresholds indicative of the flow rate of at least the OHA flowing in OHA tube 30 and intubation tube 22. Such thresholds depend on the application and implementation and may comprise, for example, the voltage ranges described above for flow regulator 36.

In some embodiments, system 11 comprises one or more displays, such as but not limited to a display 15 or a display (not shown) of control unit 33.

In some embodiments, controller 44 is configured to display, e.g., on display 15, one or more parameters indicative of the flow rate, and/or concentration of oxide and/or carbon dioxide, and/or temperature and/or humidity and/or any other suitable parameter of the OHA as well as the exhaust air flowing in tubes 30 and 32 as described above. For example, controller 44 is configured to display the oxygen concentration, humidity-level and flow rate of the OHA flowing in OHA tube 30, and the flow rate and concentration of carbon dioxide of the exhaust air flowing in exhaust tube 32.

In some embodiments, based on one or more signals received from one or more sensors 38, which are indicative of the concentration of carbon dioxide and the flow rate of the exhaust air, controller 44 is configured to switch between a normal-ventilation mode (NVM) and a hyper-ventilation mode (HVM) of ventilation system 11. In the context of the present disclosure and in the claims, the term NVM refers to about 15 breathing cycles per minute, and the term HVM refers to any suitable larger number of breathing cycles per minute, such as but not limited to, between about 100 and 300 breathing cycles per minute.

In the context of the present disclosure and in the claims, the terms "about" or "approximately" for any numerical values or ranges indicate a suitable dimensional tolerance that allows the part or collection of components to function for its intended purpose as described herein.

In some embodiments, controller 44 is configured to switch between the NVM and HVM modes by controlling the frequency at which air compressor 23 and humidifier 25 of OHAS 24 are producing the specified attributes of the OHA produced by OHAS 24.

Typically, controller 44 comprises a general-purpose controller or a general-purpose processing device, which is programmed in software to carry out the functions described herein. The software may be downloaded to the controller in electronic form, over a network, for example, or it may, alternatively or additionally, be provided and/or stored on non-transitory tangible media, such as magnetic, optical, or electronic memory.

This particular configuration of ventilation system 11 is shown by way of example, in order to illustrate certain problems that are addressed by embodiments of the present invention and to demonstrate the application of these embodiments in enhancing the performance of such a system. Embodiments of the present invention, however, are by no means limited to this specific sort of example system, and the principles described herein may similarly be applied to other sorts of ventilation systems and/or to any other sorts of ventilation subsystems used, for example, as modules of a system for conducting any medical procedure.

In other embodiments, instead of tubes 30 and 32, ventilation system 11 may have a single tube coupled to the aforementioned devices incorporated into substrate 28. In such embodiments, controller 44 is configured to calculate an integral on the flow of: (i) the OHA supplied to lungs 14 (e.g., defined as a positive flow), and (ii) the air exhaled from lungs 14, (e.g., defined as a negative flow), so as to monitor and control the tidal volume of the OHA and air in lungs 14.

### A METHOD FOR PRODUCING A VENTILATION SYSTEM

Fig. 2 is a flow chart that schematically illustrates a method for manufacturing, producing, configuring or assembling for patient use, ventilation system 11, in accordance with an embodiment of the present invention. The method begins at an air compressor and humidifier (ACH) receiving step 100, with receiving OHAS 24 having air compressor 23 and humidifier 25 described in Fig. 1 above. At a tubes receiving step 102, intubation tube 22 for insertion into trachea 12, OHA tube 30 for flowing the OHA toward lungs 14, and exhaust tube 32 for flowing exhaust air away from lungs 14, are received. At an OHA tube connecting step 104, OHA tube 30 is connected between OHAS 24 and intubation tube 22, via bifurcation adaptor 20, as described in Fig. 1 above. At an exhaust tube connecting step 106, exhaust tube 32 is connected between intubation tube 22 (via bifurcation adaptor 20) and AES 42 of the hospital described in Fig. 1 above.

At a tubes connecting step 108, tubes 18 are connected between OHAS 24 and outlets 21 of hospital supply 16 for flowing air and oxygen into OHAS 24. At a substrate receiving step 110, substrate 28, in the present example PCB substrate having electrical traces 26, is received. At a control unit production step 112, control unit 33 is produced by incorporating into substrate 28 one or more sensors 38, FSV 34, pressure regulator 35, flow regulators 36, and controller 44, as described in Fig.1 above.

At a control unit connecting step 114 that concludes the method, control unit 33 is connected with (i) tubes 30 and 32, (ii) OHAS 24 and (iii) display 15. In other embodiments, control unit 33 may comprise a display, and therefore, may not be connected to display 15.

As described in Fig. 1 above, disposable-intended (single-patient use only) components of system 11, such as but not limited to, one or more of FSV 34, pressure regulator 35 and flow regulators 36 may be used in an oxygen-enriched atmosphere after being cleaned and qualified.

In some embodiments, the method described above may comprise additional steps described herein, which has to be carried out before control unit production step 112 described above. In such embodiments, the aforementioned components are: (i) cleaned using any suitable technique, followed by (ii) applying silicon grease to predefined locations of these components, and subsequently, (iii) saturating the components with liquid oxygen, so as to clean up residues of the silicon grease and/or oil.

After concluding the additional processes described in the above paragraph, these components may undergo operative qualification testing, and subsequently, may be used as disposable components of system 11, as described in Fig.1 above.

Although the embodiments described herein mainly address ventilation systems, the methods and systems described herein can also be used in other applications, such as in medical systems having a ventilation assembly or module, and particularly in medical systems used in cardiac procedures involving patient breathing pausing.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A ventilator system for a patient, the ventilator system comprising:
a substrate, which is coupled to: (i) a first tube for flowing oxygen-enriched humidified air (OHA) toward lungs of the patient, and (ii) a second tube for evacuating exhaust air exhaled from the lungs;
one or more sensors, which are incorporated into the substrate and are configured to output a signal indicative of at least one of a flow rate, a pressure and a composition of at least one of the OHA and the exhaust air;
one or more valves, pressure regulators and flow regulators, which are: (i) incorporated into the substrate, (ii) having at least one of electromagnets and membranes, and (iii) configured to set the flow rate and the pressure of at least the OHA; and
a controller, which is configured, based on the signal, to control the one or more valves, pressure regulators and flow regulators to set at least one of the flow rate and the pressure of at least the OHA.

2. The ventilator system according to claim 1, wherein the one or more sensors comprise at least a sensor selected from a list of sensors consisting of: a flow sensor, an oxygen concentration sensor, a carbon dioxide concentration sensor, a humidity sensor, and a temperature sensor.

3. The ventilator system according to claim 1, and comprising (i) an air compressor, which is configured to compress a preassigned gas mixture of the OHA to a preassigned pressure, and (ii) a humidifier, which is configured to set a preassigned humidity of the OHA, and wherein the controller is configured, based on the signal, to control the air compressor and the humidifier by setting the preassigned gas mixture, pressure and humidity.

4. The ventilator system according to claim 3, and comprising an intubation tube, which is configured to flow the OHA and the exhaust air between the lungs and the first and second tubes, and having (i) a distal end, configured to be inserted into a trachea of the patient, and (ii) a proximal end, configured to be connected to the first and second tube, and wherein the controller is configured to control the gas mixture, the flow rate, the pressure, and the humidity of the OHA flowing into the lungs.

5. The ventilator system according to claim 1, wherein the substrate comprises a printed circuit board having electrical traces configured to electrically connect between the (i) one or more sensors, valves, pressure regulators and flow regulators, and (ii) the controller.

6. The ventilator system according to claim 1, wherein the controller is configured to display one or more parameters indicative of at least one of the flow rate and the pressure of at least the OHA.

7. The ventilator system according to claim 1, wherein at least one of the valves comprises a fail-safe valve, which is configured, in response to a failure in at least one of the flow regulators and the pressure regulators, to limit at least one of the flow rate and pressure of at least the OHA.

8. A method for producing a ventilator system for a patient, the method comprising:
providing a substrate;
coupling to the substrate:
one or more sensors for outputting a signal indicative of at least one of a flow rate, a pressure and a composition of at least one of an oxygen-enriched humidified air (OHA) flowing into lungs of the patient and exhaust air exhaled from the lungs;
one or more valves, pressure regulators and flow regulators having at least one of electromagnets and membranes for setting at least one of the flow rate and a pressure of at least the OHA; and
a controller, which is operatively connected to the valves, the pressure regulators, the flow regulators and the sensors; and
coupling the substrate to: (i) a first tube configured to flow the OHA toward the lungs, and (ii) a second tube configured to evacuate exhaust air exhaled from the lungs.

9. The method according to claim 8, and comprising (i) cleaning the one or more valves, pressure regulators, and flow regulators, (ii) applying silicon grease at one or more locations of at least one of the valves, the pressure regulators and the flow regulators, and (iii) saturating the components with liquid oxygen.

10. The method according to claim 8, wherein coupling the one or more sensors comprises coupling at least a sensor selected from a list of sensors consisting of: a flow sensor, an oxygen concentration sensor, a carbon dioxide concentration sensor, a humidity sensor, and a temperature sensor.

11. The method according to claim 8, and comprising connecting the substrate to: (i) an air compressor, for compressing a preassigned gas mixture of the OHA to a preassigned pressure, and (ii) a humidifier, for setting a preassigned humidity of the OHA.

12. The method according to claim 8, and comprising positioning an intubation tube between the lungs and the first and second tubes, the intubation tube having (i) a distal end, for insertion into a trachea of the patient, and (ii) a proximal end, for connecting to the first and second tube.

13. The method according to claim 8, wherein the substrate comprises a printed circuit board having electrical traces for electrically connecting between the (i) one or more sensors, valves, pressure regulators, and flow regulators, and (ii) the controller.

14. The method according to claim 8, wherein coupling the one or more valves comprises coupling to the substrate a fail-safe valve for limiting at least one of the flow rate and pressure of at least the OHA, in response to a failure in at least one of the flow regulators and the pressure regulators.

15. A computer software product, comprising a non-transitory computer-readable medium in which program instructions are stored, which instructions, when read by a controller for a ventilator system for a patient, the controller programmed to perform the steps of:
setting preassigned thresholds for at least one of the gas mixture, the flow rate and the humidity of an oxygen-enriched humidified air (OHA) to be directed to the lungs of the patient;
receiving one or more of the signals from sensors of the system, to control the gas mixture, the flow rate and the humidity of the OHA directed into lungs of the patient;
displaying on a display of the system, one or more parameters indicative of at least one of the flow rate,
concentration levels of the gas mixture or humidity of the OHA being directed into lungs of the patient.

16. The computer software product of Claim 15, further comprising performing the steps of:
receiving one or more of the signals from sensors of the system, to monitor expiration from the lungs of the patient.

17. The computer software product of Claim 16, further comprising performing the steps of:
setting a preassigned threshold for the temperature of the OHA to be directed to the lungs of the patient; and
receiving one or more of the signals from sensors of the system, to monitor the temperature of expiration from the lungs of the patient.

18. The computer software product of Claim 15, further comprising performing the steps of:
setting preassigned thresholds for at least one of the gas mixture having oxygen percentage between about 21% and 100%, the flow rate pressure between about 0 Cm and 100 Cm, and the humidity of the OHA between about 40% and 100%.

19. The computer software product of Claim 15, further comprising performing the steps of:
switching between a normal-ventilation mode (NVM) and a hyper-ventilation mode (HVM) for the ventilation, wherein the NVM comprises about 15 breathing cycles per minute, and
the HVM comprises between about 100 and 300 breathing cycles per minute.
